# EUROPEAN PATENT SPECIFICATION

(11) **EP 4 274 688 B1**
(45) Date of publication and mention of the grant of the patent: **30.07.2025**
(21) Application number: 22705451.7
(22) Date of filing: 07.01.2022
(51) Int. Cl.: B05B 1/00, B05B 1/26, G01M 99/00

(54) **NASAL SPRAY CONTAINER**
NASENSPRÜHERBEHÄLTER
RÉCIPIENT DE PULVÉRISATION NASALE

(30) Priority: 08.01.2021 GB 202100259
(43) Date of publication of application: 15.11.2023
(73) Proprietor: Copley Scientific Limited, Nottingham Nottinghamshire NG4 2JY (GB)
(72) Inventor: SMITH, Malcolm, Nottingham Nottinghamshire NG4 2JY (GB); SMITH, Adam, Nottingham Nottinghamshire NG4 2JY (GB); BRADLEY, Benjamin, Nottingham Nottinghamshire NG4 2JY (GB); COPLEY, Mark Andrew, Nottingham Nottinghamshire NG4 2JY (GB)
(74) Representative: Adamson Jones
(86) International application number: PCT/GB2022/000010
(87) International publication number: WO 2022/148951

(56) References cited:
- WO-A2-02/096490
- US-A1- 2020 346 236

## Description

This invention relates to a collection container. In particular, this invention relates to a collection container for use with nasal sprays, which may be used either for dose measurement or for waste disposal. See WO02/096490.

A nasal spray is a device used to deliver a medicament into the nose, in a particulate or aerosolised form. A nasal spray typically comprises a small bottle or container containing a medicament, with a depressible nozzle. The user applies a downward force to a shoulder at the base of the nozzle , which causes the release of a dose of medicament from the nozzle. To use a nasal spray, the user inserts the nozzle into a nostril and depresses the nozzle whilst inhaling to draw the medicament into the body.

It is important that the dose administered by a nasal spray is accurate and reliable, and that any waste doses can be disposed of safely. Regulatory bodies worldwide require dose content tests to be carried out on nasal sprays. This is both during product development and marketing approval, and as part of ongoing quality assurance once the product is on the market.

To comply with the USP standard for nasal sprays, USP <601>, there are a number of criteria which must be fulfilled when carrying out a dose content test. The spray should be held in a vertical or near vertical position, with the valve facing upwards. The dose should be delivered into a suitable container, from which quantitative transfer can be achieved. A suitable analytical method should be used to determine the amount of the medicament in each dose.

The problem with complying with this standard is that, when used vertically and sprayed into a suitably sized container, spray droplets rebound off the container walls or otherwise fall onto the nozzle of the spray. These droplets are not captured by the test, reducing its accuracy.

Several solutions are commonly used to overcome this problem. However, each of these solutions carries its own disadvantages. A collection container having a larger volume may be used to reduce the rebound of droplets and allow the aerosol to fully form. However, a larger container requires the use of higher volumes of dissolvents to ensure that the medicament is fully removed from all surfaces, increasing the risk of inaccuracies and the cost of the test. The nasal spray may be held in a non-vertical position to encourage falling droplets to remain within the collection container. However, this makes the test more difficult to control, and risks contravening the requirements of USP 601. Finally, a seal may be used around the nozzle to reduce the surface area exposed to rebounding and/or falling droplets. However, this increases the risk of contamination from particles from previous actuations on the nozzle, as well as increasing the complexity and cost of the container itself.

The scintillation vials which are commonly used for collection have a volume of 10ml, which is far larger than the typical dose volume (0.1ml), which further makes it difficult to recover all of the dose from the container.

Another aspect of nasal sprays is the need to dispose safely of waste doses (where drug recovery is not required). This requires the collection and disposal of high volumes of waste doses with no splash-back onto the nozzle, and safe, convenient disposal of the collected doses.

There is now provided the following invention which overcomes and/or substantially mitigates the aforementioned and/or other problems associated with the state of the art.

There is provided a dose collection container as defined in the appended claim 1. Further optional features are recited in the associated dependent claims.

The dose collection container comprises at least two diverging impact surfaces that direct droplets from the nasal spray along an internal surface of the collection container, preventing the droplets from rebounding onto the nozzle. This is relevant to the collection of a medicament both for dosage measurement, and for waste disposal.

Deflecting droplets across an internal surface of the container retains the droplets inside the device, rather than allowing them to fall onto the nozzle, or out of the aperture. This increases the accuracy of dosage measurement carried out using the container of the invention.

With regard to waste management, the effective retention of the medicament inside the container through the use of divergent impact surfaces increases safety, reducing or preventing contamination of the surrounding area.

The design does not require additional parts such as seals, which means it can be manufactured from a single material, creating a cost effective but effectual solution.

The dose collection container of the invention comprises first and second opposing walls. The dose collection container may comprise one or more additional walls which extend between the first and second opposing walls. Thus, the first and second opposing walls may form the base wall and the ceiling wall of the container, while the one or more additional walls form one or more sidewalls of the container, defining a void in the interior of the container for the collection of a nasal spray dose. Alternatively, the internal surfaces of the first and second walls may abut one another, with a void between the two defined by the divergent impact surfaces.

The container may be any suitable shape. Externally, the container may have the shape of a cylinder, a cuboid, or other prism. The external shape of the container may preferably be a cylinder. The first and second walls may have an internal surface and an external surface, wherein the internal surface is located on the interior of the dose collection container, and the external surface is located on the exterior of the dose collection container.

The dose collection container may have an internal volume which makes recovery and measurement of the dose content easier. Typically, this may be achieved by having a smaller internal volume than a typical scintillation vial. Thus, the internal volume of the dose collection container may be less than 10ml, or may be less than 5ml. The internal volume of the dose collection container may be from about 0.1ml to about 10ml, or from 0.5ml to about 5ml, or from 0.5ml to about 4ml. The small internal volume is enabled by the divergent impact surfaces; as these surfaces direct spray along an internal surface it is not necessary to rely on a large internal surface area to disperse the droplets and prevent rebound onto the nozzle. This leads to more accurate measurement, and particularly improves performance when measuring small doses of medicament.

The internal volume of a dose collection container intended for waste disposal may be smaller than the internal volume of a dose collection container intended for dosage content measurement. The internal volume of a dose collection container intended for waste disposal may be from 0.5ml to 2ml, or from 0.5ml to 1.5ml, or may preferably be about 1.1ml. The internal volume of a dose collection container intended for dosage content measurement may be from 2ml to 4ml, or from 2.5ml to 3.5ml, or may preferably be about 2.8ml.

The dose collection container may be formed in multiple parts such that, when separated, the internal surfaces of the dose collection container can readily be accessed to aid in recovery of the medicament for measurement. The dose collection container may be formed in two sections. The two sections of the dose collection container may be releasably connected together, eg by a screw-fit, a snapfit, or a friction-fit joint. After a nasal spray dose has been injected into the dose collection container, the two sections may be separated to permit easy recovery of the medicament dose. The dose collection container may comprise first and second sections, wherein the first section comprises the first wall and the second section comprises the second wall. Complementary formations about the periphery of the first and second sections may engage to releasably connect the two sections. For example, a ridge around the periphery of the first section may engage with a complementary shoulder about the periphery of the second section, the ridge and shoulder engaging with each other in a friction fit.

Thus, the dose collection container may comprise a first section and a second section, the first and second sections being releasably connected together.

Alternatively, the dose collection container may be a single unit which cannot be opened. This may be particularly appropriate where the dose collection container is to be used for waste disposal, where recovery of the medicament is not required.

The first wall comprises an aperture sized to receive a nozzle of a nasal spray. The aperture should be large enough that at least a portion of the nozzle may be accommodated within the aperture. Once inserted into the aperture, the sides of the nozzle may abut the aperture about the circumference of the nozzle. Alternatively, and preferably, the nozzle may be positioned in the aperture such that it does not contact the aperture, or the collection container, to reduce the risk of crosscontamination.

The aperture may be circular. The aperture may be centrally positioned in the first wall. The walls of the aperture may extend in a line perpendicular to the external surface of the first wall, or may be angled relative to a line perpendicular to the external surface of the first wall. In particular, the walls of the aperture may be angled such that the diameter of the aperture on the internal face of the first wall is smaller than the diameter of the aperture on the external face of the first wall. Thus, the aperture may have a frusto-conical cross-sectional shape. The aperture may have a diameter on the external face of the collection container of between 10mm and 15mm, or of between 12mm and 15mm, or of between 13mm and 14mm. The aperture may have a diameter on the internal face of the collection container of between 5mm and 10mm, or of between 6mm and 9mm, or of between 7mm and 8mm. This frusto-conical shape provides a closer fit between the nozzle and the walls of the aperture, reducing or preventing the loss of medicament.

The aperture may further comprise means for guiding the nozzle into the aperture, reducing the precision required when inserting the nozzle. Thus, the aperture may further comprise one or more guides, or walls, which may contact the nozzle during insertion and lead it into the aperture. The guide may take the form of an annular wall, surrounding the aperture and extending outwardly from the first wall. At least an internal surface of the annular wall may be angled such that the interior shape of the guide has a frusto-conical cross-section.

The internal surface of the second wall comprises at least two diverging impact surfaces which, on actuation of a nasal spray into the dose collection container, deflect the spray along an internal surface. This minimises the risk of rebound, and encourages any drips that form to run to the sides of the container. The second wall may comprise two, or three, or four, or five, or more diverging impact surfaces which deflect the spray along an internal surface. The at least two diverging impact surfaces may extend outwardly from the second wall, or may comprise depressions formed in the surface of the second wall. The depressions may be circular, or may be elliptical, when seen in a plan view.

A point located centrally on an internal surface of the second wall forms a central point. A first line extending through the central point, in the same plane as the second wall, forms a first axis. A second line, extending through the central point in a direction perpendicular to the second wall (and perpendicular to the first axis) forms a second axis. Each diverging impact surface extends outwardly from the first axis. An acute angle may be formed between each divergent surface and the second axis. The acute angle may be formed between each divergent surface and the second axis either above or below the first axis, but preferably above the first axis, such that at least a portion of each divergent surface extends away from the first wall. The acute angle formed between each divergent surface and the second axis may be 1-89°, or may be 10-80°, or may be 20-70°, or may be 30-60°.

A ridge is located in the plane defined by the first axis. The ridge may be straight, such that it is parallel to or extends along the first axis, or may be curved or arcuate, forming a convex or concave shape. The ridge forms the line at which the at least two diverging impact surfaces meet. The ridge may extend across the width of the second wall, or may extend across only a portion of the second wall. The ridge may extend across a central portion of the second wall, and each end of the ridge may end in a planar surface. The ridge may be vertically offset from the surface of the second wall, that is, the ridge may be formed in a depression in the second wall.

Thus, the ridge may form an arcuate, concave curve in the second wall, extending across a central portion of the second wall. The two ends of the arcuate ridge, each of which may comprise a planar surface, may protrude from the second wall, such that they extend towards, but do not contact, the first wall. This arcuate shape encourages any droplets which may form on the internal surface of the dosage collection container to run to the end of the ridge, rather than to rebound or drip onto the surface of the nozzle.

The at least two diverging impact surfaces may be planar, or curved. Each diverging impact surface may comprise two or more discrete facets, or may comprise a single continuous surface. Each diverging impact surface may comprise a planar region and a curved region, or two planar regions, or two curved regions.

Each diverging impact surface may comprise a continuous arcuate surface. The continuous arcuate surface may form a concave depression in the second wall defining an arc which extends from a proximal end at which it contacts the second axis to a distal end at which it contacts the surface of the second wall, or an internal edge of the second wall, or a sidewall of the container. The distal end of the arc may be located at an edge of the concave depression in the second wall, or at the edge of the concave depression which is located furthest from the first axis, measured along a line perpendicular to the first axis. The proximal end of the arc may be located at a point on the ridge. The proximal end of the arc may be vertically offset from the distal end of the arc, such that the proximal end of the arc is located between 1 and 15 mm, or between 5 and 12 mm, or between 6 and 10 mm, above the distal end of the arc, when measured vertically (wherein the external side of the second wall is considered to be the top of the dosage collection container). The arc may be a regular arc. The arc may be an irregular arc, wherein the apex of the arc is located off-centre along a line perpendicular to the second axis, and extending between the second axis and the distal end of the arc. The perpendicular distance between the second axis and the apex of the arc may be smaller than the perpendicular distance between the apex of the arc and the distal end of the arc.

Each diverging impact surface may comprise a planar region and a curved region. In this embodiment, the general shape of the diverging impact surface may follow the same description as described above in relation to a continuous arcuate surface, except that the portion of the diverging impact surface located either before or after the apex may be planar. Thus, the diverging impact surface may comprise a planar portion extending from a proximal end located on the first axis, from the central point or from a ridge, to the apex, and a curved portion extending from the apex to a distal end located on the surface of the second wall, or an internal edge of the surface of the second wall, or a sidewall of the dose collection container. Alternatively, a curved portion may extend from the proximal end to the apex, and a planar portion extends from the apex to the distal end. The perpendicular distance between the proximal end and the apex may be smaller than the perpendicular distance between the apex and the distal end.

The at least two diverging impact surfaces may be symmetrical about the first axis, and/or may be symmetrical about a ridge extending in the plane formed by the first axis.

The void in which the nasal spray is collected may be formed between the first and second walls. At least a portion, for example at least the periphery of the internal surfaces of the first and second walls may abut one another, such that the void is formed by the diverging impact surfaces in the second wall. The internal surface of the first wall may be substantially planar, while the internal surface of the second wall comprises concave depressions which form the at least two diverging impact surfaces, as described above. The internal surfaces of the first and second walls may substantially abut one another, such that a cavity is formed by the void between the planar first wall and the at least two diverging impact surfaces in the second wall.

Where the dose collection container is to be used for waste disposal, the container may further comprise an exit port configured for connection to a vacuum pump. Connection to a vacuum pump enables the dose collection container to be continuously evacuated, and waste material disposed of safely. The vacuum pump may comprise a cyclone, wherein air carrying the waste medicament is drawn into the pump through an inlet, and pass through a waste vessel connected to the vacuum pump. Droplets in the waste air stream are captured in the waste vessel, which can be removed for safe disposal of the collected droplets.

There is thus provided a vacuum pump for the disposal of waste medicament, wherein the vacuum pump is configured for connection to a dose collection container and to a waste vessel and wherein, upon actuation of the vacuum pump, air is drawn from the dose collection container and passes through the waste vessel, and wherein waste medicament present in the air stream is deposited in the waste vessel.

The dose collection container may be used by hand, that is, the nasal spray and dose collection container may be held by hand, the nozzle inserted into the aperture in the first wall, the nasal spray actuated and subsequently removed from the dose collection container by the user.

A holder may be provided to facilitate handheld use of the dose collection container, which may aid with gripping the container, aligning the nasal spray with the dosage container, ensuring that an even force is applied to the nasal spray, and/or holding the nozzle in position such that it does not contact the dose collection container (eg it does not contact the aperture during actuation). The holder may be configured such that a downward force is applied to the shoulders which extend outwardly from the base of the nozzle, rather than to the nozzle itself, creating a more even force and more consistent dispensing of the medicament.

The holder may comprise an elongate container for receipt of the nasal spray. The nozzle of the nasal spray may protrude from a first end. An abutment surface or surfaces may protrude from an internal surface of the holder which, in use, engage the shoulders extending from the base of the nozzle on the nasal spray. When a downward force is applied to the holder, the abutment surface engages the shoulders of the nasal spray and applies a downward force, actuating the nasal spray. The dose collection container described herein may be placed on the nozzle of the nasal spray prior to actuation, for collection of the dispensed dose. The holder may further comprise a support surface which engages a surface of the dose collection container to retain it in place. The support surface may comprise an end wall, or an edge of an end wall, of the holder.

The holder may be designed to be used by hand, or may be configured for attachment to an actuation device, eg for use with the Vertus range of testing devices manufactured by Copley Scientific Limited. Thus, the holder may comprise structural features which enable it to be coupled to an actuation device. For example, the holder may comprise a stepped ring or a clip which mates with a complementary ring or clip on the actuation device, ensuring that the centre of the nozzle is aligned with the opening. The dose collection container itself may alternatively comprise the structural features necessary to connect it to an actuation device.

The use of different holders which provide any specific structural features required for handheld use or use with an actuation device permits a single dose collection container to be produced which may be used in both methods.

The use of a holder may also reduce or remove the need for a lead-in or guide surface to guide the nozzle into the aperture. The holder ensures that the nozzle is guided accurately and correctly into the opening, at the optimum depth, and ensures that forces are correctly distributed to prevent tilting of the nozzle during actuation. When used with an actuator device, force is applied to the actuator rather than to the nozzle, which prevents movement of the nozzle and keeps the forces in line with the nasal spray actuator body.

Described herein is a holder configured to retain a nasal spray, the nasal spray having one or more shoulders located at the base of a nozzle, the interior of the holder comprising an abutment surface which, in use, engages the one or more shoulders of the nasal spray and, upon the application of a downward force, causes actuation of the nasal spray.

The dose collection container may be made from any suitable material which is dimensionally stable and has good chemical resistance to organic drug recovery sovlents. Particularly suitable materials include 316 stainless steel, aluminium titanium, polyether ether ketone, nylon and acetal. Preferably, the dose collection container is made from 316 stainless steel.

The at least two divergent surfaces in the second wall may be formed on a lathe. The component may be inserted into the lathe such that it is offset from its central axis, to enable a first divergent surface to be turned. The component is then offset from the central axis in the opposite direction to enable a second divergent surface to be turned.

Alternatively, the at least two divergent surfaces in the second wall may be milled using computer numerical coding (CNC) and an appropriate cutter, eg a bullnose cutter.

The dose collection container may alternatively be 3D printed. This may be particularly appropriate where the device is to be used as a waste collection container, and recovery of the medicament is not required.

Embodiments of the invention will now be illustrated by the following figures:
Figure 1 is a perspective view of the underside of a dosage collection container according to a first embodiment of the invention, in which the dosage collection container comprises two releasably connectable sections;
Figure 2 shows the dosage collection container of Figure 1, wherein the two releasably connectable sections are separated;
Figure 3a is a line drawing showing a side view of the dosage collection chamber of Figure 1;
Figure 3b is a cross-sectional view of the dosage collection chamber of Figure 3a along the line B-B;
Figure 4 is a cutaway view of one section of the dosage collection chamber of Figure 1, showing the divergent impact surfaces;
Figure 5 is a perspective view of the underside of a dose collection container according to a second embodiment of the invention;
Figure 6a is a plan view of the topside of the dose collection container of Figure 5;
Figure 6b is a cross-sectional view of the dose collection container of Figure 6a along the line B-B
Figure 7a is a side view of a holder to facilitate hand held collection of a nasal spray dose in a dose collection container;
Figure 7b is a cross sectional view of a holder to facilitate hand held collection of a nasal spray dose in a dose collection container; and
Figure 8 is a perspective view of a vacuum pump for use in connection with a dose collection container.

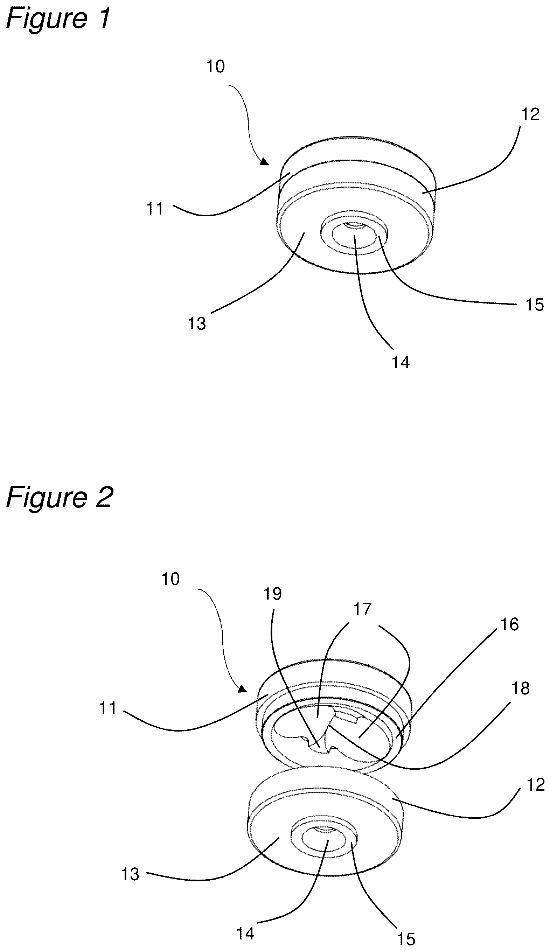

A dosage collection container 10 according to a first embodiment of the invention is shown in Figure 1. The dosage collection container comprises first and second releasably connectable sections 11, 12, which are shown connected by a friction fit in Figure 1 and separated in Figure 2. The dosage collection container 10 comprises a first wall 13 in which is formed a circular aperture 14, the aperture 14 having a guide 15 to facilitate the insertion of a nozzle. The guide 15 takes the form of an annular wall extending outwardly from the dosage collection container about the periphery of the aperture 14.

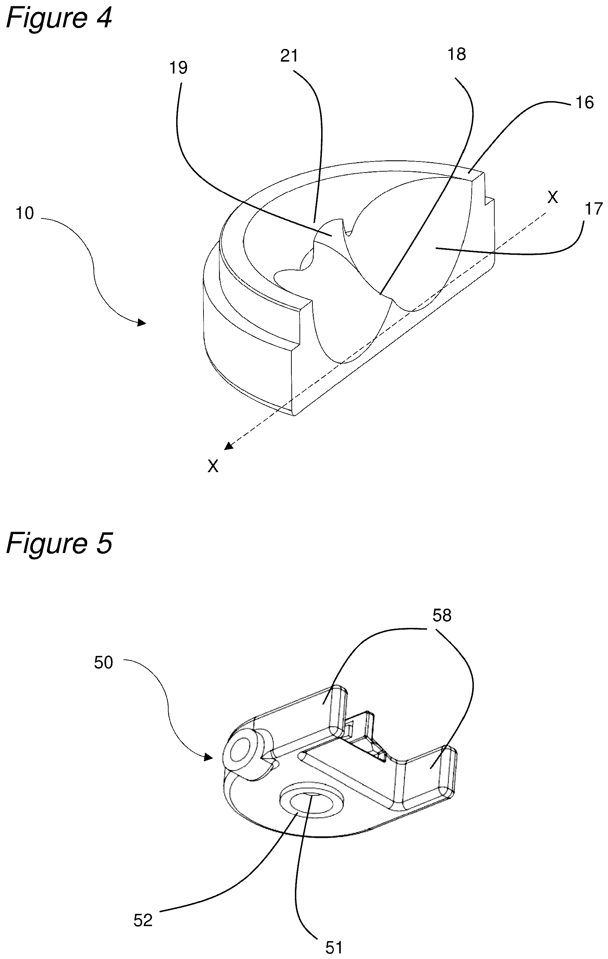

The dosage collection container further comprises a second wall 16. The internal surface of the second wall 16 which opposes and abuts the first wall 13 in the dosage collection container can be seen in Figure 2. When the two sections 11, 12 are connected, the first wall 13 and the second wall 16 abut each other about a periphery of the second wall 16. The interior surface of the second wall 16 comprises two divergent impact surfaces 17 which meet at an arcuate ridge 18 extending along a portion of a line extending through a central point on the second wall 16. Each end of the arcuate ridge 18 opens into a planar surface 19, which protrudes from the surface of the second wall and encourages droplets to flow away from the centre of the second wall. The divergent impact surfaces 17 comprise concave depressions in the internal surface of the second wall 16, and can be more clearly seen in Figure 4. The divergent impact surfaces 17 each define an arc extending from a proximal end at which it contacts the ridge 18 to a distal end at which it reaches the internal surface of the second wall 16 and, when the two sections 11, 12 are connected, contacts the first wall 13 of the dosage collection container. The arc described is visualised along the line X-X as shown in Figure 4.

The distal end of the arc defined by the divergent impact surface is vertically offset from the proximal end.

In use, the nozzle of the nasal spray is inserted into the aperture 14 and the spray actuated. Any droplets which contact the divergent impact surfaces 17 will be directed along an internal surface of the container 10, preventing rebound of the droplets onto the nozzle and increasing the accuracy of the dosage measurement, as well as removing the need for a tight seal to be formed between the nozzle and the walls of the aperture 14.

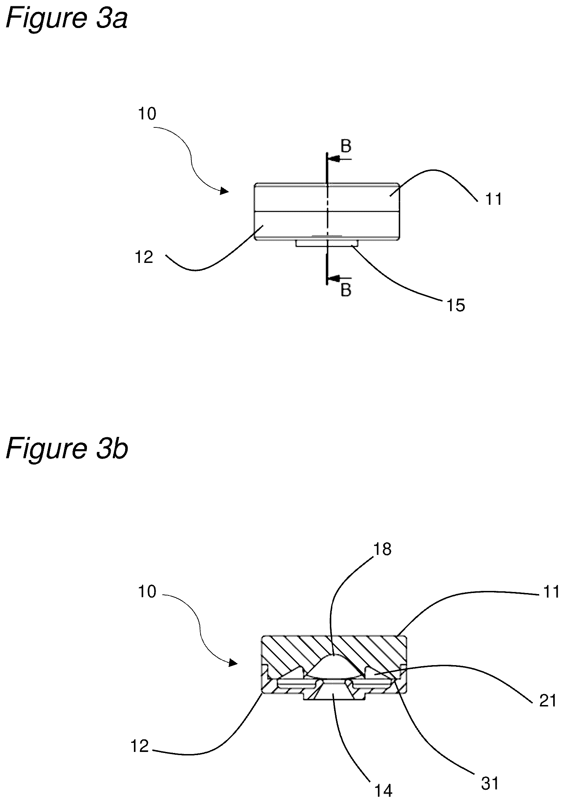

Figure 3a is a line drawing showing the embodiment of Figure 1, and Figure 3b is a cross-sectional view of Figure 3a along the line B-B (along the line of the ridge 18 in Figure 2). This cross-sectional view shows the frusto-conical shaped aperture 14, and an end view of one divergent impact surface 17. The divergent impact surface 17 forms a concave depression in the second wall 16. Additional triangular shaped depressions 21 are present at each side of the ridge 18, formed by the protrusion of the ends of the arcuate ridge 18 beyond the surface surface of the second wall. The first and second walls abut each other about their periphery 31, such that the void for collection of a nasal spray dose is formed by the concave depressions in the surface of the second wall.

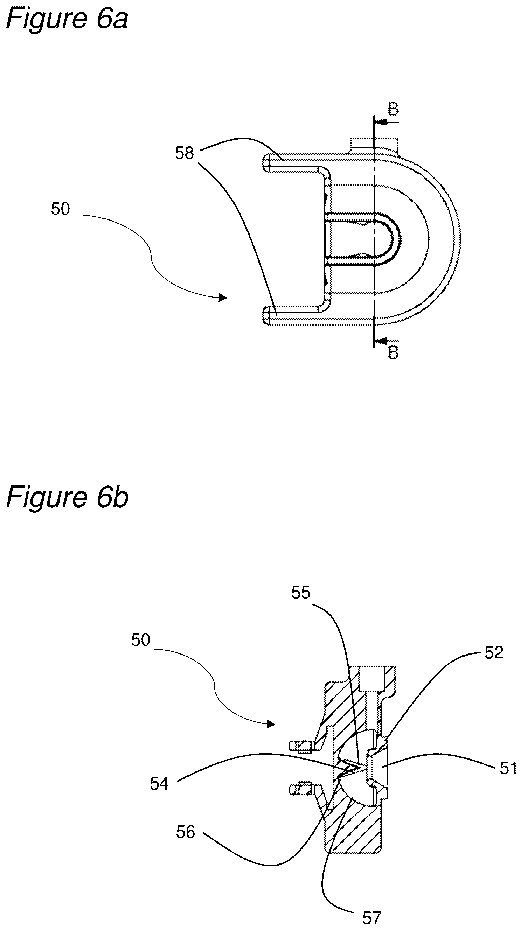

A dosage collection container 50 specifically configured for use as a waste collection container can be seen in Figures 5-7. This collection chamber cannot be readily opened, as there is no requirement to recover the dosage. The collection container 50 comprises a frusto-conical shaped aperture 51 with a guide 52 to facilitate insertion of the nozzle. Divergent impact surfaces 53 on an opposing wall each comprise a planar surface 54 extending from a central ridge 55 to an apex 56, and a curved surface 57 extending from the apex 56 to the first wall of the container, the void being defined between the first wall of the container and the divergent impact surfaces.

The waste collection container 50 comprises arms 58 for easy connection to an actuation device, such as the Vertus range of testing devices manufactured by Copley Scientific Limited.

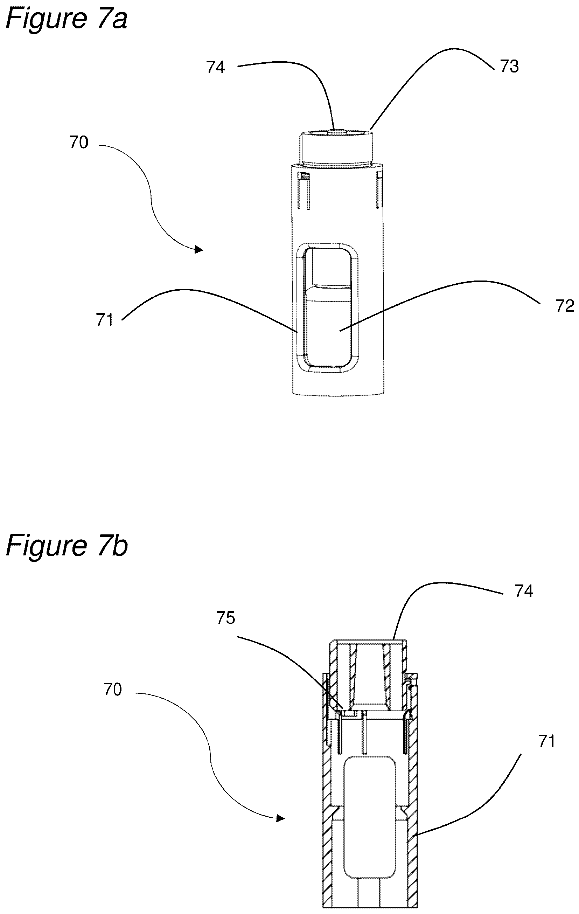

A holder 70 to facilitate handheld collection of a nasal spray dose in a dose collection container can be seen in Figures 7a and 7b. The holder comprises a body 71 having a cavity into which the nasal spray 72 can be inserted, the nozzle 73 of the nasal spray protruding from one end of the holder, and an end wall 74 of the holder providing a surface on which the dose collection container can be located. An abutment surface 75 abuts the neck of the nasal spray, retaining it in place within the holder.

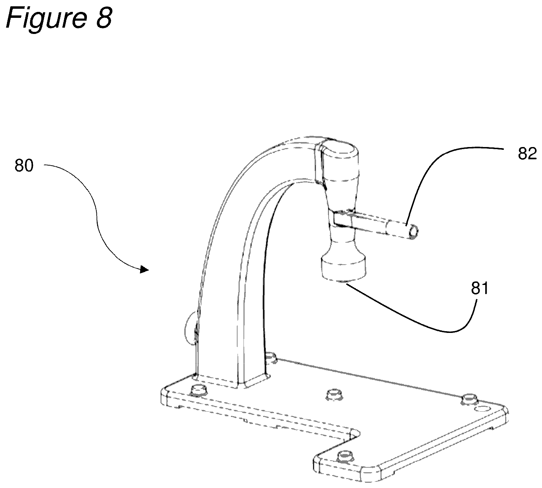

The dose collection container described herein may be used to collect waste doses of medicament, and a vacuum pump 80 such as that shown in Figure 8 may be connected to a port on the dose collection container for this purpose. A waste vessel (not shown) is connected to the vacuum pump 80 at the point designated 81. Air carrying the waste medicament is drawn from the dose collection container (not shown) through the inlet 82, and is drawn down into and up out of the waste vessel, where droplets from the air stream are deposited. The waste bottle can be safely and conveniently removed for disposal of the waste medicament.

## Claims

1. A dose collection container (10) comprising first and second opposing walls (13,16), the first wall (13) comprising an aperture (14) sized to receive a nozzle of a nasal spray, and the second wall (16) comprising at least two diverging impact surfaces (17) which, on actuation of a nasal spray into the dose collection container (10), deflect the spray along an internal surface, **characterised in that** a first axis extends through a central point on the internal surface of the second wall (16), in the same plane as the internal surface of the second wall (16), and a ridge (18) is located in the plane defined by the first axis, wherein the at least two diverging impact surfaces (17) extend outwardly from the ridge (18).

2. A dose collection container (10) according to Claim 1, which has an internal volume of less than 10 ml.

3. A dose collection container (10) according to any preceding claim, further comprising a second axis extending through the central point in a direction perpendicular to the first axis, wherein an acute angle is formed between each diverging impact surface (17) and the second axis.

4. A dose collection container (10) according to any preceding claim, wherein the ridge (18) is arcuate.

5. A dose collection container (10) according to Claim 3, wherein the at least two diverging impact surfaces (17) are curved, wherein each curved divergent impact surface (17) defines an arc extending from a proximal end at which it contacts the second axis to a distal end at which it contacts the surface of the second wall (16), such that each curved divergent impact surface (17) forms a concave depression in the second wall (!6).

6. A dose collection container (10) according to Claim 5, wherein the apex of the arc is located closer to the proximal end than the distal end, when measured along a line perpendicular to the second axis, and/or wherein the proximal end is vertically offset from the distal end.

7. A dose collection container (10) according to Claim 3, wherein each of the at least two diverging impact surfaces (17) comprises a planar region and a curved region.

8. A dose collection container (10) according to Claim 7, which comprises a planar portion extending from a proximal end located in the plane formed by the first axis to an apex, and a curved portion extending from the apex to a distal end located on the surface of the second wall (16), forming a concave depression in the surface of the second wall (16).

9. A dose collection container (10) according to Claim 8, wherein the distance between the proximal end and the apex is smaller than the distance between the apex and the distal end, when measured along a line perpendicular to the second axis.

10. A dose collection container (10) according to any preceding claim, wherein the at least two diverging impact surfaces (17) are symmetrical about the first axis.

11. A dose collection container (10) according to any preceding claim wherein the first wall (13) comprises an internal surface and an external surface, and the second wall (16) comprises an internal surface and an external surface, wherein the at least two diverging impact surfaces (17) are located on the internal surface of the second wall (16), and wherein at least a portion of the internal surface of the first wall (13) abuts at least a portion of the internal surface of the second wall (16).

12. A dose collection container (10) according to Claim 11, wherein the diverging impact surfaces (17) form a void between the opposing first and second walls (13.16).

13. A dose collection container (10) according to any preceding claim, which is configured for use with an actuation device.

14. A kit of parts comprising a dose collection container (10) according to any preceding claim and a holder (70), the holder (70) being configured to connect the dose collection container (10) to an actuation device.

## Patentansprüche

1. Dosissammelbehälter (10), umfassend eine erste und eine zweite Wand (13,16), die sich sich gegenüberliegen, wobei die erste Wand (13) eine Öffnung (14) umfasst, die bemessen ist, um eine Düse eines Nasensprühers aufzunehmen, und die zweite Wand (16) mindestens zwei divergierende Aufprallflächen (17) umfasst, die bei Betätigung eines Nasensprühers in den Dosissammelbehälter (10) hinein das Spray entlang einer Innenfläche ablenken, **dadurch gekennzeichnet, dass** sich eine erste Achse durch einen zentralen Punkt auf der Innenfläche der zweiten Wand (16) in der gleichen Ebene wie die Innenfläche der zweiten Wand (16) erstreckt und sich eine Rippe (18) in der Ebene befindet, die von der ersten Achse definiert ist, wobei sich die mindestens zwei divergierenden Aufprallflächen (17) von der Rippe (18) nach außen erstrecken.

2. Dosissammelbehälter (10) nach Anspruch 1, der ein Innenvolumen von weniger als 10 ml aufweist.

3. Dosissammelbehälter (10) nach einem vorhergehenden Anspruch, ferner umfassend eine zweite Achse, die sich durch den zentralen Punkt in einer Richtung senkrecht zu der ersten Achse erstreckt, wobei ein spitzer Winkel zwischen jeder divergierenden Aufprallfläche (17) und der zweiten Achse gebildet ist.

4. Dosissammelbehälter (10) nach einem vorhergehenden Anspruch, wobei die Rippe (18) bogenförmig ist.

5. Dosissammelbehälter (10) nach Anspruch 3, wobei die mindestens zwei divergierenden Aufprallflächen (17) gekrümmt sind, wobei jede gekrümmte divergierende Aufprallfläche (17) einen Bogen definiert, der sich von einem proximalen Ende, an dem sie die zweite Achse berührt, zu einem distalen Ende erstreckt, an dem sie die Oberfläche der zweiten Wand (16) berührt, sodass jede gekrümmte divergierende Aufprallfläche (17) eine konkave Vertiefung in der zweiten Wand (16) bildet.

6. Dosissammelbehälter (10) nach Anspruch 5, wobei sich der Scheitel des Bogens näher zu dem proximalen Ende als dem distalen Ende befindet, wenn entlang einer Linie senkrecht zu der zweiten Achse gemessen, und/oder wobei das proximale Ende vertikal von dem distalen Ende versetzt ist.

7. Dosissammelbehälter (10) nach Anspruch 3, wobei jede der mindestens zwei divergierenden Aufprallflächen (17) einen planaren Bereich und einen gekrümmten Bereich umfasst.

8. Dosissammelbehälter (10) nach Anspruch 7, der einen planaren Abschnitt, der sich von einem proximalen Ende, das sich in der von der ersten Achse gebildeten Ebene befindet, zu einem Scheitel erstreckt, und einen gekrümmten Abschnitt, der sich von dem Scheitel zu einem distalen Ende, das sich auf der Oberfläche der zweiten Wand (16) befindet, erstreckt und eine konkave Vertiefung in der Oberfläche der zweiten Wand (16) bildet, umfasst.

9. Dosissammelbehälter (10) nach Anspruch 8, wobei der Abstand zwischen dem proximalen Ende und dem Scheitel kleiner als der Abstand zwischen dem Scheitel und dem distalen Ende ist, wenn entlang einer Linie senkrecht zu der zweiten Achse gemessen.

10. Dosissammelbehälter (10) nach einem vorhergehenden Anspruch, wobei die mindestens zwei divergierenden Aufprallflächen (17) symmetrisch um die erste Achse sind.

11. Dosissammelbehälter (10) nach einem vorhergehenden Anspruch, wobei die erste Wand (13) eine Innenfläche und eine Außenfläche umfasst und die zweite Wand (16) eine Innenfläche und eine Außenfläche umfasst, wobei sich die mindestens zwei divergierenden Aufprallflächen (17) auf der Innenfläche der zweiten Wand (16) befinden, und wobei mindestens ein Abschnitt der Innenfläche der ersten Wand (13) an mindestens einem Abschnitt der Innenfläche der zweiten Wand (16) anliegt.

12. Dosissammelbehälter (10) nach Anspruch 11, wobei die divergierenden Aufprallflächen (17) einen Hohlraum zwischen der ersten und der zweiten Wand (13,16), die sich gegenüberliegen, bilden.

13. Dosissammelbehälter (10) nach einem vorhergehenden Anspruch, der zur Verwendung mit einer Betätigungsvorrichtung konfiguriert ist.

14. Kit von Teilen, umfassend einen Dosissammelbehälter (10) nach einem vorhergehenden Anspruch und einen Halter (70), wobei der Halter (70) dazu konfiguriert ist, den Dosissammelbehälter (10) mit einer Betätigungsvorrichtung zu verbinden.

## Revendications

1. Récipient de collecte de dose (10) comprenant des première et seconde parois opposées (13,16), la première paroi (13) comprenant une ouverture (14) dimensionnée de manière à recevoir une buse d'un pulvérisateur nasal, et la seconde paroi (16) comprenant au moins deux surfaces d'impact divergentes (17) qui, lors de l'actionnement d'une pulvérisation nasale dans le récipient de collecte de dose (10), dévie la pulvérisation le long de la surface interne, **caractérisé en ce qu'**un premier axe s'étend à travers un point central sur la surface interne de la seconde paroi (16), dans le même plan que la surface interne de la seconde paroi (16), et une arête (18) est située dans le plan défini par le premier axe, lesdites au moins deux surfaces d'impact divergentes (17) s'étendant vers l'extérieur à partir de l'arête (18).

2. Récipient de collecte de dose (10) selon la revendication 1, qui présente un volume interne inférieur à 10 ml.

3. Récipient de collecte de dose (10) selon une quelconque revendication précédente, comprenant en outre un second axe s'étendant à travers le point central suivant une direction perpendiculaire au premier axe, un angle aigu étant formé entre chaque surface d'impact divergente (17) et le second axe.

4. Récipient de collecte de dose (10) selon une quelconque revendication précédente, ladite arête (18) étant arquée.

5. Récipient de collecte de dose (10) selon la revendication 3, lesdites au moins deux surfaces d'impact divergentes (17) étant incurvées, chaque surface d'impact divergente incurvée (17) définissant un arc s'étendant depuis une extrémité proximale au niveau de laquelle elle est en contact avec le second axe à une extrémité distale au niveau de laquelle elle est en contact avec la surface de la seconde paroi (16), de sorte que chaque surface d'impact divergente incurvée (17) forme une dépression concave dans la seconde paroi (16).

6. Récipient de collecte de dose (10) selon la revendication 5, ledit sommet de l'arc étant situé plus près de l'extrémité proximale que de l'extrémité distale, lorsqu'il est mesuré le long d'une ligne perpendiculaire au second axe, et/ou ladite extrémité proximale étant décalée verticalement par rapport à l'extrémité distale.

7. Récipient de collecte de dose (10) selon la revendication 3, chacune desdites au moins deux surfaces d'impact divergentes (17) comprenant une zone plane et une zone incurvée.

8. Récipient de collecte de dose (10) selon la revendication 7, qui comprend une partie plane s'étendant depuis une extrémité proximale située dans le plan formé par le premier axe jusqu'à un sommet, et une partie incurvée s'étendant depuis le sommet jusqu'à une extrémité distale située sur la surface de la seconde paroi (16), en formant une dépression concave dans la surface de la seconde paroi (16).

9. Récipient de collecte de dose (10) selon la revendication 8, ladite distance entre l'extrémité proximale et le sommet étant inférieure à la distance entre le sommet et l'extrémité distale, lorsqu'elle est mesurée le long d'une ligne perpendiculaire au second axe.

10. Récipient de collecte de dose (10) selon une quelconque revendication précédente, lesdites au moins deux surfaces d'impact divergentes (17) étant symétriques par rapport au premier axe.

11. Récipient de collecte de dose (10) selon une quelconque revendication précédente, ladite première paroi (13) comprenant une surface interne et une surface externe, et ladite seconde paroi (16) comprenant une surface interne et une surface externe, lesdites au moins deux surfaces d'impact divergente (17) étant situées sur la surface interne de la seconde paroi (16), et au moins une partie de la surface interne de la première paroi (13) étant en appui sur au moins une partie de la surface interne de la seconde paroi (16).

12. Récipient de collecte de dose (10) selon la revendication 11, lesdites surfaces d'impact divergentes (17) formant un vide entre les première et seconde parois opposées (13,16).

13. Récipient de collecte de dose (10) selon une quelconque revendication précédente, qui est conçu pour être utilisé avec un dispositif d'actionnement.

14. Kit de pièces comprenant un récipient de collecte de dose (10) selon une quelconque revendication précédente et un support (70), le support (70) étant conçu pour raccorder le récipient de collecte de dose (10) à un dispositif d'actionnement.
